# EUROPEAN PATENT APPLICATION

(11) **EP 4 176 803 A1**
(43) Date of publication of application: **10.05.2023**
(21) Application number: 21206807.6
(22) Date of filing: 05.11.2021
(51) Int. Cl.: A61B 5/024, A61B 5/0245, A61B 5/26, A61B 5/28, A61B 5/332, A61B 5/363, A61B 5/361, A61B 5/00

(54) **PORTABLE ECG DEVICE**

(71) Applicant: Anidium Limited, Cambridge CB1 2LA (GB)
(72) Inventor: DROKOV, Igor, Cambridge (GB)
(74) Representative: Gill Jennings & Every LLP

(57) **Abstract**

A portable device configured to measure activity of a user's heart, the device comprising: a sensor configured to measure the electrical signals of a user's heart, the sensor comprising: a first electrode and a second electrode, the first and second electrodes are configured to come into contact with first and second parts of a user's skin, wherein the first and second electrodes are spaced apart from each other and located on opposite sides of the portable device; an activation switch configured to activate the sensor; wherein the activation switch is configured to activate the sensor through a single interaction with the activation switch by the user; wherein once the sensor has been activated, the device is configured to record the measured signals in a memory of the device; and wherein the sensor is activated for a pre-determined period of time.

## Description

### Field of the Invention

The present invention relates to portable devices for measuring the activity of a user's heart, in particular measuring an electrocardiogram (ECG) of a user.

### Background of the Invention

An electrocardiogram (ECG) is a simple test that can be used to check the heart's rhythm and electrical activity by recording the electrical signals in your heart. Sensors in the form of a plurality of electrodes are attached to the skin which are able to detect the electrical signals produced by your heart each time it beats. The signals are recorded and then analysed by a doctor to see if they are unusual. A series of ECGs can also be taken over time to monitor a person already diagnosed with a heart condition or taking medication known to potentially affect the heart.

An ECG is a quick means of detecting heart problems and monitoring heart health. They can be used to investigate symptoms of a possible heart problem, such as chest pain, palpitations (suddenly noticeable heartbeats), dizziness and shortness of breath. ECGs can help detect arrhythmias, coronary heart disease, heart attacks, and cardiomyopathy.

Atrial Fibrillation (AF) is the most common cause of an irregular heartbeat and a leading cause of stroke. It is estimated that early detection and treatment of AF avert one stroke in every 25 patients. AF detection has been prioritised across healthcare systems worldwide.

There are two main challenges with AF detection: firstly asymptomatic patients and the need for comprehensive and affordable routine screening solutions; and secondly patients presenting with symptoms of palpitations and the need to capture an ECG at the time symptoms occur. This is especially difficult to achieve in patients whose symptoms are infrequent and irregular. Current standard of care (SOC) includes conducting a 12-lead-test at a surgery or a 24-48 Hour Holter monitor test (often repeated). These tests have limitations for patients with infrequent symptoms, who require longer periods of monitoring.

Given the seriousness of heart conditions, it is important that these are detected as early as possible. It is also important to quickly rule out the possibility of a serious heart condition (which may put a patient's mind at ease) so that other possible medical conditions can be investigated. Allowing a patient to monitor their own heart health may help reduce diagnosis time.

A number of options are emerging which provide the patient with the ability to carry out an ECG without the need to go to a hospital. One option includes ECG patches which are provided to a patient as a chest patch that can be worn typically for up to 14 days continuously recording ECG data. A problem with this option, is that the typical monitoring and recording duration (e.g. 14 days) is not a sufficient length of time to capture the ECG during the symptoms onset for patients with intermittent symptoms and so the only option for such patients is to re-administer the test, which is often not economically feasible. The patch also relies on sticking to the user's skin using an adhesive, and this is often not effective or suitable for parts of the body typically covered in hair such as the chest. In addition, movement of the user can agitate the patch, causing it to fall off. A user's sweat may also affect the ability of the patch to stick to the user's skin. A further downside of using patches in that once they have been applied to a patient, the patches capture data continuously (there is no on/off button). This means that the patches capture data all the time through a range of activities carried out by the patient (e.g. physical exercise) and so muscle movement may affect the ability of the patches to capture reliable data. The user also isn't paying attention and trying to capture best ECG possible for the analysis/diagnosis. Furthermore, continuous capture of data means that there is a lot more data to be reviewed and analysed at the end of the monitoring period, most of this data being of low clinical relevance.

Another option includes wearable devices such as smart watches. These devices typically require some form of user interface or software, such as an app, in order to start the ECG recording and provide feedback to the user. These devices often also require regular charging, meaning that the feasibility and usability of the device for long term ECG recording is limited. Since these devices are patient operated, via an app, and are multi-functional with several buttons, there is a risk of the patient pressing the wrong buttons or not understanding any instructions or feedback they are given by the app or smart device. Therefore the user may not be able to gain instantaneous access to ECG recording functionality and miss potentially rare symptoms onset. These devices are also generally quite expensive as their primary function is not recording ECGs (e.g. smart watches) and so they have lots of extra functionality which is not needed for ECG recording.

Thus, although alternative remote monitoring devices (wearable patches and handheld monitors) are now available they are limited due to their cost and usability.

It would be advantageous to provide a device which can measure an ECG of a user, which does not suffer from at least the above problems.

### Summary of the Invention

According to a first aspect there is provided a portable device configured to measure activity of a user's heart. The device comprises a sensor configured to measure the electrical signals of a user's heart. The sensor comprises a first electrode and a second electrode, the first and second electrodes are configured to come into contact with first and second parts of a user's skin, wherein the first and second electrodes are spaced apart from each other and located on opposite sides of the portable device. The portable device also comprises an activation switch configured to activate the sensor. The activation switch is configured to activate the sensor through a single interaction with the activation switch by the user. Once the sensor has been activated, the device is configured to record the measured signals in a memory of the device. The sensor is activated for a pre-determined period of time.

The portable device measures electrical signals of a user's heart and so the portable device which is able to measure an ECG of a user. The activation switch acts as a one-touch button that can be used to activate the device, making the device quick and simple to use. By providing an activation switch that can be activated through a single interaction by a user, the user is able to activate the device substantially instantaneously and so the device can be used to measure electrical signals from the heart as soon as symptoms appear. This provides better clinical utility. Additionally, by providing a single activation switch, the user does not have to worry about incorrectly activating the device, for example by pressing the wrong button. Furthermore, the portable device is operable without the need to interact with additional devices such as mobile devices or computing devices. That is, the device is activated by the activation switch alone and does not require software to activate the device. This avoids lengthy set-up times for the device, enabling the user to quickly activate the device as soon as they need to.

The device is also configured to record the measured electrical signals once the sensor has been activated. In this way, the activation switch has the effect of initiating the measuring and the recording, and so can be considered as having dual functionality. The recording is therefore initiated only when measurements are being taken, which helps avoid unnecessary recording of data which may fill up the device memory. The measuring and the recording may be initiated substantially simultaneously to ensure that all the measured data is captured and recorded by the device.

Activating the sensor for a pre-determined period of time means that discrete measurements, and thus discrete recordings, are taken rather than continuous measurements and recordings. This ensures that the device only captures data when the user decides to capture data, for example when symptoms start to show, rather than capturing all data relating to electrical heart signals all the time.

The portable device may be configured to record measured signals on-demand. In other words, the device does not continuously record data, but only when required by the user. In particular, the device only records data when the user has activated the device using the activation switch. In this way, the device can be considered as being in a passive state, such as a sleep mode, before the device has been activated. This may help conserve power allowing the device to be used for long periods of time. The measured signals may comprise a plurality of discrete measurements. Thus, the device measures and records multiple data sets.

Preferably, the first electrode forms part of the activation switch. This may simplify the device making the device easy to use by a user. This may also make the device more compact as there may be fewer individual components, which helps ensure the device is discreet.

Preferably, the first electrode is located on a top surface of the device and the second electrode is located on a bottom surface of the device. A top surface is a surface of the portable device that is furthest away from the user when the device worn. A bottom surface is a surface of the portable device that is closest to the user when the device is worn. This arrangement of electrodes may allow the portable device to be worn at multiple locations on a user so that electrical heart signals can be measured at multiple different sites.

The first electrode may be temporarily deformable. Thus, the first electrode may return to its original shape after it has been deformed for example through interaction with the first electrode by a user when activating the sensor using the activating switch.

Preferably, the first electrode is made of flexible material. Flexible material may allow the first electrode to temporarily deform. The first electrode may be made of a thin sheet of stainless steel. The second electrode may be made from a sheet of stainless steel. Stainless steel may provide better quality signal from the sensor as well as being a material that is biocompatible with users.

The portable device may comprise a housing configured to house electrical components of the portable device. This may protect the electrical components of the device from becoming damaged.

Preferably, the first electrode is integrally formed with the housing. In this way, the housing and the first electrode may be considered as a single component. This reduces the overall number of components in the device, which reduces the manufacturing costs. A smaller number of components also reduces the overall size of the device, helping provide and compact, and therefore discreet, device. The first electrode may be considered as being sealed within the housing. This may help provide a water resistant device because this arrangement avoids the need for a join, and therefore a gap in which water can enter, between the housing and the first electrode.

In some examples, the second electrode may be detachable from the portable device. In this way, the second electrode can be removed which may provide access to internal components, such as electrical circuitry or batteries, for replacement or maintenance.

Preferably the second electrode comprises a raised portion. The raised portion may be configured to come into contact with a user's skin. The raised portion may provide better electrical contact between the user and the device. In particular, the raised portion may provide better electrical contact between the user and the device compared to a flat second electrode.

Preferably, the first and second electrodes are electrically connected to a PCBA via at least one flexible connection. The flexible connection may be flexible in height such that in a first position the flexible connection has a first height and in a second position the flexible connection has a second height. Preferably, there are a plurality of flexible connections. A first flexible connection may connect the first electrode to the PCBA and a second flexible connection may connect the second electrode to the PCBA. The at least one flexible connection provides a flexible electrical connection between the electrodes and the PCBA which helps ensure there is a good electrical contact between the electrodes and the PCBA. Preferably, the at least one flexible connection is a resilient connection. In this way, the connection may return to its original shape after it has been deformed. This may allow the connection to be compressed and return to its original shape after a compression force is removed, without damaging the electrical connection. In some examples the at least one flexible connection may be at least one spring.

In some examples, the activation switch comprises a feedback mechanism such as a tactile switch. The feedback from the feedback mechanism may inform the user that they have pressed the activation switch and that they have activated the device. The tactile switch may provide direct feedback to the user in the form of tactile feedback. A tactile switch is a discreet mechanism for providing feedback to the user about whether an action, such as activating the device, has been performed as it does not make a noise that can be heard by others. Tactile feedback also does not require the user to be looking at the device to see if they have activated the device meaning that the user can discretely activate the device, for example under a table.

The portable device may further comprise an indication means configured to provide information to a user about the status of the device. For example the indication means may indicate to a user when power is low or when the device has been activated.

Preferably, the indication means may comprise a form of light. The light may be an LED or an OLED, or any other suitable form of lighting. A light, such as an LED is a simple means of providing information quickly to a user. A light may also be a discreet means of providing status information to a user because lights such as LEDs are typically small in size and they do not make a noise. A light can also be viewed by a person with limited eyesight compared to text-based indication means which require a user to focus on a screen which may include small detail.

The device may comprise an interface configured to allow the user to interact with the device. Preferably, the interface only includes the activation switch and the indication means. More preferably, the interface only includes a single activation switch and a single indication means in the form of a single light such as a single LED. In this way, the device interface is minimal. This simple interface provides the user with a limited way in which the user can interact with the device and a limited way in which the user can receive information from the device. The simple interface allows the device to be used by anyone without the need to learn any new skills, for example how to operate new buttons or functions. The minimal interface also does not comprise any text-based features and so users with poor, or extremely limited, eyesight are still able to make full use of the device. Providing a simple interface also ensures the device remains simple and easy to use, by a user, in stressful situations such as when symptoms occur. In this case, the user is able to fully interact with the device without the risk of pressing the wrong buttons or starting an incorrect operation.

The portable device may be configured to be held against the user when measuring the electrical signals of a user's heart such that the first part of the user's skin is located on the user's hand and the second part of the user's skin is located anywhere on the user. For example, the first part may be on the finger and the second part may be on the chest, either leg, or either arm. In this way, the device can be applied to multiple locations on the body in order to record the electrical signals of the heart. In other words, the device is not limited to use at one particular location. As such, the device may be considered a multi-site device. This allows the device to capture multiple different and equivalent ECG leads using single device, and so a large amount of data can be captured using a single, rather than multiple devices. The device is arranged to be temporarily held in a particular position against a user's skin when a measurement is being taken and recorded by the sensor. In this way, the device is not in permanent contact with the user' skin, which may become uncomfortable or cause irritation.

Preferably, the portable device is configured to be held against the user when measuring the electrical signals of a user's heart such that the first part of the user's skin is located on the user's hand and the second part of the user's skin is located on the back of the user's wrist. Here the back of the wrist is the underside of the wrist. As the back of the user's wrist is relatively small in size, the device must also be small so that it fits on this site and non-intrusive. This site on the user's body is also easy to access, facilitating use of the device. This area also has little to no hair, in general, providing good electrical contact between the user and the device. Further, the back of the wrist also does not require any preparation before the device can be used, for example the area does not need drying or shaving to remove hair, and so the device can be used instantly in this location.

The portable device may comprise an attachment means configured to allow the user to wear the device. This allows the user to wear the device without holding the device. In some examples, the attachment means may comprise a strap, for example a wrist strap or a chest strap. In other examples, the attachment means may comprise a keyring or lanyard attachment loop. Providing an attachment means reduces the likelihood of the user losing the device.

The attachment means may be configured to retain the sensor in contact with a part of the user's skin. For example, the attachment means may be a wrist strap which holds the sensor in contact with a user's wrist. This may ensure that a good contact is made between the sensor and the user's skin, so that signal quality is improved. This may also reduce the need for the user to retain the sensor in pace themselves while measurements are being taken, which may result in lower quality signals.

Preferably, the portable device comprises a memory configured to store the electrical signals measured by the sensor. The memory is preferably an internal memory. The memory may store the electrical signals in the form of data. The memory may be configured to store multiple instances of measuring a user's electrical heart signals. This may allow the user to wear the device over a period of time and collect multiple measurements over this period of time without the need to remove the device. This allows the user to put on the portable device and forget about it once it is being worn.

The electrical signals may be measured over a pre-defined time period. The observation period and frequency of recordings may be pre-selected by a medical professional, and programmed into the portable device, in accordance with an observation plan suitable for a particular user. The device may therefore be tailored to each user.

The portable device may further comprises a removable battery. This may prolong the useful life of the portable device. Preferably the battery is a user-replaceable battery. In this way, a user is able to replace the battery themselves when the battery runs out rather than requiring a specialist with particular technical knowledge or technical skills to replace. In this way, the user can replace the battery themselves using common tools that they may have available to them, such as a screwdriver. Preferably, the battery is a single-use battery such as a single-use coin cell battery. A problem with rechargeable batteries is that when a device comprising a rechargeable battery is being charged, the device is no longer portable or wearable because it is plugged in. Also, devices comprising rechargeable batteries generally do not function until a minimum level of charge has been reached, and so the device is non-functioning for a period of time. These problems are solved by providing a single-use replaceable battery. In this way, the user can replace the battery as soon as needed and they do not have to wait for the device to charge up. Instead, the device becomes immediately available to be used again as soon as the battery has been changed.

The single interaction may comprise a single press of the activation switch. This is a quick and simple means of activating the portable device.

The portable device may be a multi-use, multi-user device configured to allow recorded measured signals captured by one user to be read and then deleted from the device and then issue the device to another user to allow the device to be used by multiple users at different points of time.

Advantageously, a multi-use, multi-user device allows the device to be used by a first user to take one or more readings which can later be downloaded onto a computing device and stored in relation to that user. The device can then be sanitised and re-issued to a second user to take measurements from the second user. In this way, a single device can be used to record multiple ECG tests to multiple users over the lifetime of the device.

Preferably, the portable device is able to be used by multiple users, and so may be referred to as a multi-user device. This may be achieved by providing a read-write memory within the device. In this way, the device can be used by multiple users by deleting records of a first user so that records for a second user can be stored within the memory. Furthermore the portable device is a standalone device in that it does not require any software or an app on a computing device to activate the device. This means that the device is not associated with a particular user profile. Instead the device can be passed between multiple users which each have the ability to activate the device so that the device can record ECGs of many different users. The device may also be a multi-use device rather than a single-use device. In other words, the device can be applied to a user's skin and removed, and then subsequently re-applied after a simple sanitisation step to another area of either the same user's skin or a different user's skin.

Generally, the portable device enables any patient at risk to undertake an affordable AF test at the convenience of their home and provide their GP with a clinically actionable report. The portable device is a completely remote, end to end, cost effective solution.

The wearable ECG device allows a patient to instantly record ECGs at the time symptoms occur by pressing the device and maintaining contact with skin for at least 30 seconds over a period of a few weeks. This allows an ECG to be captured immediately at the time symptoms appear which can be used to quickly produce a diagnosis.

The patient is therefore actively involved in monitoring their condition through at-home monitoring that does not require unnecessary visits to GP surgeries. No technical expertise is needed to operate the device.

### Brief Description of the Drawings

Embodiments of the invention will now be described by way of example only with reference to the accompanying drawings in which:
Figures 1A, 1B, and 1C show perspective views of a portable device;
Figure 2 shows part of a portable device;
Figures 3A and 3B show exploded views of a portable device;
Figure 4 shows a cross-section of part of a portable device;
Figure 5 shows a cross-section of part of a portable device;
Figures 6A, 6B, 6C show parts of a portable device;
Figure 7 shows a cross-section of part of a portable device; and
Figure 8 illustrates a method of using the device.

### Detailed Description

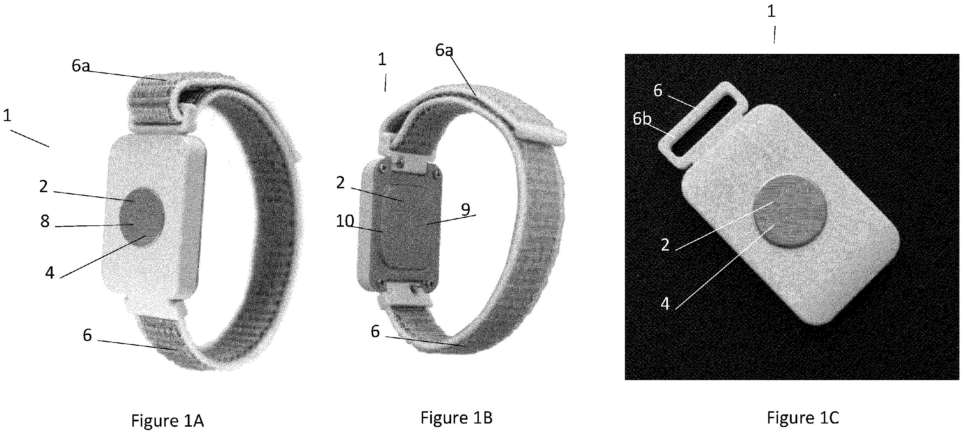

Figure 1A shows an example device 1 configured to measure activity of a user's heart, and in particular measure an ECG. The device comprises a sensor 2 configured to measure the electrical signals of a user's heart and an activation switch 4 configured to activate the sensor 2. The activation switch 4 is configured to activate the sensor 2 through a single interaction with the activation switch 4 by the user, for example by pushing the activation switch 4. The device 1 is a portable device allowing the device to be picked up and carried by a user.

In the illustrated examples, the device 1 further comprises an attachment means 6 configured to allow the user to wear the device 1 about their person. As shown in Figures 1A and 1B, the attachment means 6 can take the form of a wrist strap 6a, for example a Velcro strap, to allow the user to wear the device 1 on their wrist. In other examples, such as those illustrated in Figure 1C, the attachment means 6 can take the form of a loop 6b on the device 1, allowing a lanyard to be attached to the wearable device via the loop 6b so that the user can wear the device 1 round their neck. In some examples, the device 1 does not comprise an attachment means. In this case, the user simply picks up the device and holds it next to themselves when using the device 1.

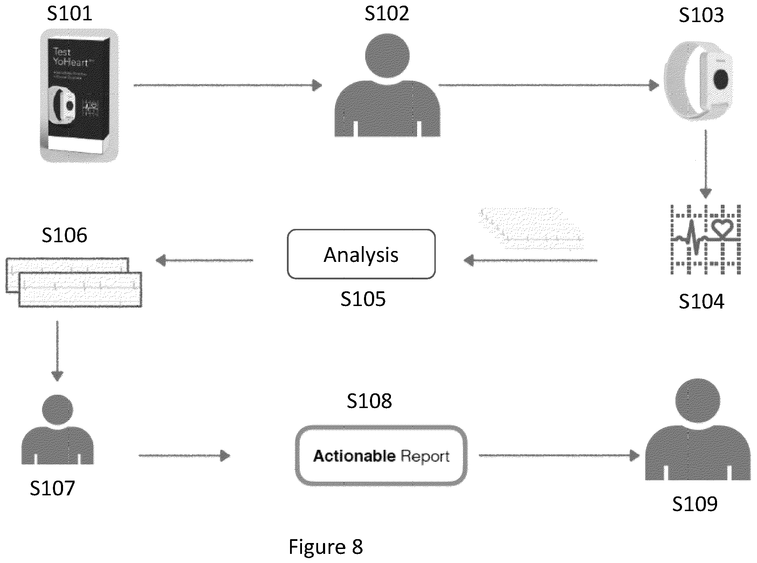

In general, and with reference to Figure 8, a medical professional (such as a doctor) provides a portable device 1 to a user (S101), who may also be referred to as a patient, who wears the device 1 in order to capture a plurality of ECG measurements over a period of time (S102), after the device 1 has been activated. For example, the device 1 may capture measurements over a period of 30 seconds, although in some cases the period of time may be greater. The ECG measurements are stored in a memory within the device 1 in the form of ECG data. The memory is able to store multiple ECG measurements captured over a period of time, in the form of a plurality of discrete data sets. The portable device 1 is returned by the patient (S103) to the medical professional at the end of a period of monitoring (for example, several days, weeks, or months). The device 1 is connected to a computing device (S104) and the ECG data is transferred from the portable device 1 to the computing device for analysis (S105). The ECG data can be presented as ECG traces (S106). The ECG is interpreted by the medical professional (S107) and a report of the results is prepared (S108). The report can then be provided back to the referring physician for further action (S109).

Further details of the portable device 1 will now be described.

The sensor 2 comprises a first electrode 8 and a second electrode 10. The first and second electrodes 8, 10 are configured to come into contact with first and second parts of a user's skin in order to measure the electrical signal of the user's heart. The first and second electrodes 8, 10 are spaced apart from each other and are located on opposite sides of the portable device 1, as shown in Figures 1A and 1B. In particular, the first electrode 8 is located on a top surface, and may be referred to as a top electrode, and the second electrode 10 is located on a bottom surface and may be referred to as a bottom electrode. Here, a top surface of the portable device 1 is a surface of the portable device 1 that is furthest away from the user when the device 1 worn, and may also be referred to as an outer surface. A bottom surface of the portable device 1 is a surface of the portable device 1 that is closest to the user when the device is worn 1, and may also be referred to as an inner surface. The first electrode 8 forms part of the activation switch 4.

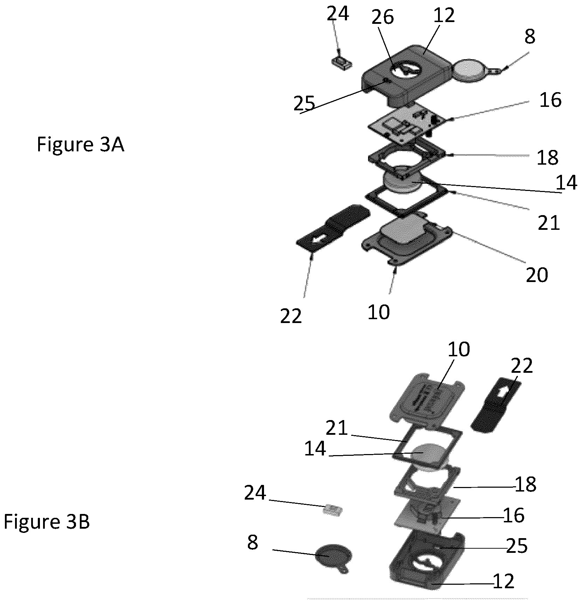

Figures 3A and 3B show exploded views of the portable device 1. The view in Figure 3A is a top-down exploded view and the view in Figure 3B is a bottom-up exploded view. Here a top-down view, is a view looking from the top surface, through the device, to the bottom surface. Similarly, bottom-up view is a view looking from the bottom surface, through the device, to the top surface.

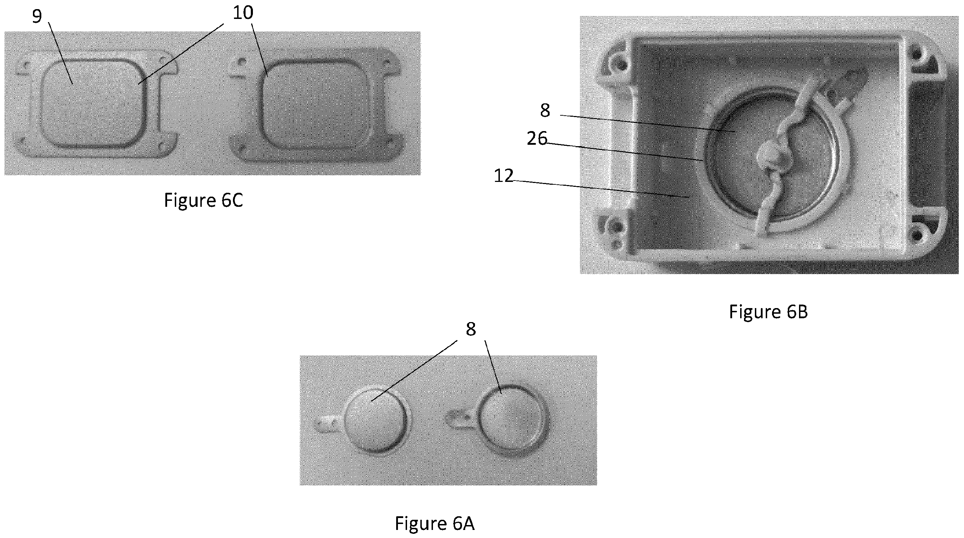

As shown in Figure 3A and 3B, the top surface of the portable device 1 takes the form of part of a housing 12, which may be made from plastic or any other suitable electrically insulating material. The bottom surface of the portable device 1 is provided by the second electrode 10, which can be seen in Figure 6C. The second electrode 10 is attached to the housing via suitable fixing means such a screws. The housing 12 creates an internal cavity in which various electronic components are housed, as seen in Figures 3A and 3B.

As shown in Figures1B and 6C, the second electrode comprises a raised portion 9 which can be located substantially centrally within the second electrode 10. The second electrode 10 therefore has a profile having a raised portion rather than a flat profile. The raised portion 9 provides an area of the second electrode 10 which has particularly good electrical contact with the user's skin. When the user presses the device 1 against their skin, the device 1 will tend to "sink" into the user. Having a raised portion 9 on the second electrode 10 means that when the device is pressed into the user, only the raised portion 9 will sink into the user and the rest of the device 1 will lie flat against the user. Conversely, if the whole second electrode was flat, when the user presses the device against themselves, the whole device 1 sinks into the user which can result in the edges of the device causing discomfort and pain to the user. By providing a raised portion 9, the hard edges of the device 1 do not get pressed into the user when the device 1 is pressed against the user during usage, which provides a more comfortable user experience or the user.

Within the housing 12 there is a battery 14 and the first electrode 8, both of which are in electrical communication with a printed circuit board assembly (PCBA) 16 which is located between the battery 14 and the first electrode 8. The battery 14 is supported by a battery bracket 18, having a hole in which the battery 14 is positioned, to retain the battery in place. An insulator sheet 20 is provided between the battery 14 and the second electrode 10, so that the battery is electrically insulated from the second electrode 10. This helps ensure that the battery 14 does not interfere with readings of the user's electrical signals taken using the second electrode 10 which is part of the sensor 2. The battery 14 is a replaceable battery, increasing the usable lifespan of the portable device 1. The battery 14 can be replaced by removing the second electrode 10, which can be done by the user themselves.

As the second electrode 10 is removable, it is important to ensure that fluids such as water do not enter the portable device 1 through the join between the second electrode 10 and the housing 12, so that the portable device 1 remains water resistant. In order to prevent the ingress of fluids a seal 21, for example a rubber seal, is provided between the second electrode 10 and the housing 12, as shown in Figures 3A and 3B. The seal 21 therefore seals the portable device 1 against water and other fluids.

Providing a water proof device also allows the device to be sanitized between uses. This is important as it allows the device to be used by multiple patients, the device being sanitized between different patients. In this way, the device is reusable across a larger number of different users which can provide significant cost savings compared to devices which can only be used by a single patient.

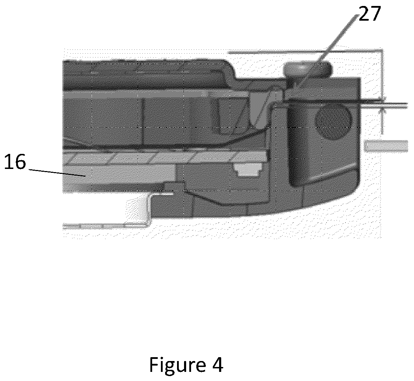

The portable device 1 also comprises a battery tab 22 which is located between the battery 14 and the PCBA 16. The battery tab 22 isolates the battery 14 from the PCBA 16 preventing the battery 14 from powering the device 1. The battery tab 22 is pulled out from the portable device 1 through a slot in the housing 12, and discarded, before the portable device 1 can be used for the first time. In order to ensure the battery tab slot in the housing 12 does not provide an entry point for water, dust, or other debris, after the battery tab 22 has been removed, a previously compressed O-ring 27 decompresses and fills in the space in the slot left behind by the battery tab 22, as shown in Figure 4.

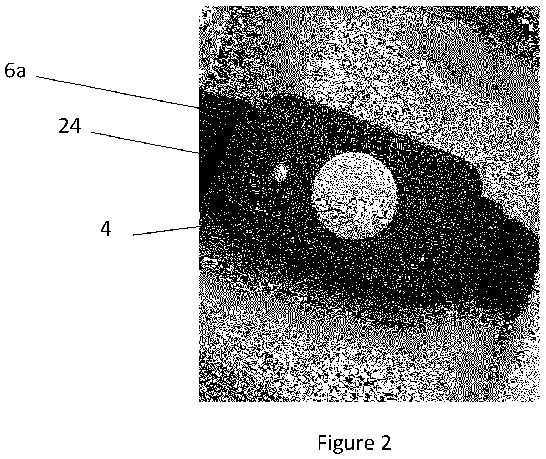

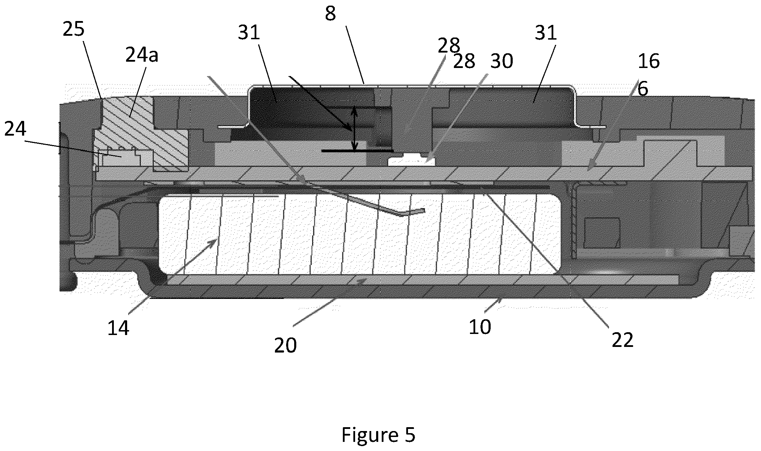

The portable device also comprises an indication means 24, in the form of a light 24 housed within the housing 12. In particular, as shown in Figure 5, there is light window 24a which covers the light 24 which in this case is an LED 24. As shown in Figures 3A, an aperture 25 in the housing 12 allows the LED 24 to be visible through the housing 12. This is also illustrated in Figure 2. The indication means 24, in this case the LED 24, can be used to provide information to the user about the status of the device. For example, the indication means 24 may be used to indicate that the device is on and measuring an ECG, or that the internal memory is full. This may be achieved, for example, using different colours or a series of one or more blinks to indicate different statuses of the portable device 1. As the portable device 1 does not comprise a display screen, an LED is a simple means of communicating information to a user without the need for a display, which would increase the overall size of the device.

The LED 24 may form part of an interface, along with the activation switch 4, that is located within the housing 12. The interface allows the user to interact with the device. As the interface only includes the LED 24 and the activation switch 4, in particular the first electrode 8, the interface is minimal with limited functionality. This ensures that the device is straightforward to operate by a user.

The limited interface of the portable device 1, having only a single activation switch 4 and a single indication means 24, helps provide a discreet device 1 which does not have a large, illuminated display or make noises. This is important for users who need to wear the device but do not want others knowing that they need to wear the device 1. The compact and narrow profile of the device also improves the overall discreetness of the device.

Figure 5 shows the internal structure of the portable device 1 in more detail. The housing 12 comprises a switch aperture 26, located substantially centrally within the top surface of the housing 12. The first electrode 8 protrudes through this switch aperture 26 so that the user can interact with the first electrode 8.

The first electrode 8 is over-moulded into the housing 12, as shown in Figure 6B. The housing 12 and the fist electrode 8 therefore form a single component. This helps reduce the overall number of parts which helps reduce manufacturing costs. This arrangement with fewer separate parts also helps reduce the overall size of the device. This is important in order to provide a discreet portable device 1 which can be worn by the user without drawing attention to the user. Another advantage of over-moulding the first electrode 8 into the housing 12 is to ensure that the first electrode 8 is sealed within the housing 12, so that it is securely held in position. Sealing the first electrode 8 within the housing also ensures there are no gaps between the components which helps ensure that the portable device 1 is water resistant. Any gaps between components which may occur when separate components are joined together, provide potential pathways for fluids such as water, or dirt, to enter the portable device 1 which could damage the internal electronics and components.

As mentioned previously, the first electrode 8 forms part of the activation switch 4. The activation switch 4 further comprises a pin 28 and a feedback mechanism 30 which is in the form of a tactile switch 30. As shown in Figure 5, the tactile switch 30 is positioned on the PCBA 16 and the pin 28 is located between the first electrode 8 and the tactile switch 30, and is in contact with both the first electrode 8 and the tactile switch 30. To activate the portable device 1, the user pushes on the first electrode 8, which is exposed to the user through the aperture 26 in the housing 12. Pushing on the first electrode 8 causes the first electrode 8 to bend slightly under the pressure which causes the pin 28 to be pushed down which in turn pushes down on the tactile switch 30. The tactile switch 30 is turned on and the portable device 1 begins measuring the electrical signals of the user's heart. As such, the pressure on the first electrode 8 is transferred into movement of the tactile switch 30 on the PCBA 16 via the pin 28. The tactile switch 30 provides positive feedback to the user, in the form of tactile feedback, so that the user knows they have successfully activated the portable device 1 using the activation switch 4.

In order to transfer the movement from the first electrode 8 via the pin 28 to the tactile switch 30, the first electrode 8 must be flexible such that it is able to temporarily deform when pressed and return to its original shape after it has been pressed. The first electrode 8 is therefore made from a flexible material for example a very thin sheet of stainless steel as shown in Figure 6A. The amount of flex in the metal used for the first electrode 8 must be sufficient to allow movement so that the activation switch 4 can be activated but not so great that a dent is formed in the first electrode 8 when pressed by a user. To help prevent the first electrode from permanently deforming, the housing 12 may comprise a plurality of ribs 31 which support part of the first electrode 8, as shown in Figure 5.

It is also important to ensure that the PCBA 16 does not flex too much when the user presses on the activation switch 4. Support for the PCBA 16 is provided by the insulator sheet 20, which has sufficient thickness to prevent the PCBA 16 from deforming when the activation switch 4 is pressed. In some examples, the insulator sheet 20 may have a thickness of 0.4 mm.

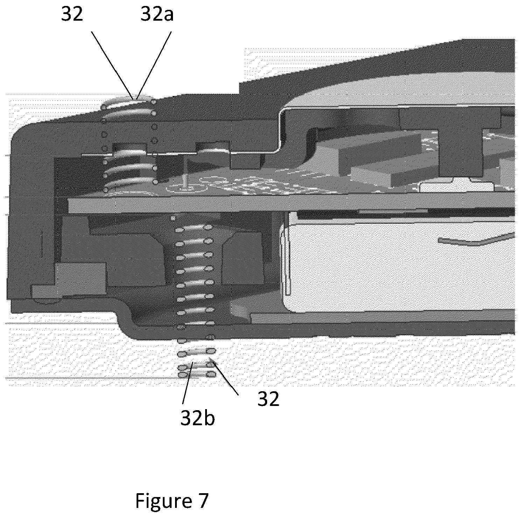

In order for the portable device 1 to function, the first and second electrodes 8, 10 must be electrically connected to the PCBA 16. This is achieved by at least one flexible electrical connection 32. In the example illustrated in Figure 7 there are two flexible electrical connections 32 which take the form of two springs 32. Thus, the electrical connections are provided by a plurality of springs 32. In particular, a first spring 32a is used to electrically connect the first electrode 8 to the PCBA 16 and a second spring 32b is used to electrically connect the second electrode 10 to the PCBA 16. The springs 32 provide a reliable connection between the electrodes 8, 10 and the PCBA 16. The springs 32 also provide a flexible contact between the electrodes 8, 10 and the PCBA 16. The flexibility means that the springs 32 allow for an easier fit between the components, whilst still ensuring good electrical contact, because the tolerances do not need to be as tight as would be required for rigid contacts.

The portable device 1 is preferably configured to be worn on the inner part of a user's wrist, as shown in Figure 2, for example by provided a wrist strap. The inner part of the wrist provides a good contact point between the device 1 and the user's skin as this area typically does not have much body hair, thus leading to better signal quality.

Due to the arrangement of the two electrodes 8, 10 on a top surface and a bottom surface of the portable device 1, the portable device 1 is able to be placed at multiple different sites on the body to record an ECG of the user at multiple different sites. In particular, the portable device 1 is able to provide a connection between a user's arm and any other second site, depending on where they place the portable device (for example chest, leg, other arm). As such, the portable device 1 is able to capture ECG signals equivalent to traditional lead placements such as limb leads and chest leads. While the portable device 1 itself is in the form of a single-lead ECG monitor, the device 1 is suitable for operation on different body sites to produce traces equivalent of other ECG leads and so the portable device 1 enables capturing multiple ECG leads from multiple points on the body. The device may therefore be considered as a multi-use device rather than a single-use device.

The single activation switch 4 provides a one-touch button which can be used to activate the device 1. This makes the device simple and easy to use as the user does not have to worry about pressing the wrong button or understand functionality of multiple buttons. By providing a single activation switch 4, which can activate the device 1 through a single interaction with the activation switch 4, it is not possible for the user to press the wrong button to activate the device. A single, one touch activation switch 4 is also quick to use. Activation of the device 1, via the activation switch 4, is substantially instantaneous after the device has been activated, meaning that an ECG can be captured exactly when symptoms occur. In other words, there is no significant or noticeable delay between a user activating the device 1 and the device recording the electrical signals of the user's heart. This allows more accurate measurement to be taken, and so a more accurate diagnosis can be made by a medical professional, leading to more accurate treatment plans for the patient.

As well as activating the sensor 2, the activation switch 4 also causes the device 1 to record the data measured by the sensor in the device memory. Thus, the activation switch 4 initiates both the measuring of the data and the recording of the data. The user therefore only has to press one switch 4 to initiate both operations. The sensor is activated for a pre-determined period of time, and so the device can be thought of as measuring and recording discreet, rather than continuous, data sets. The device 1 therefore only captures data when required by the user, for example when symptoms start to show, rather than capturing data all the time.

The single, one touch activation switch 4 is simple to operate and avoids the need for a medical professional to operate the device 1. Due to the arrangement of the first and second electrodes 8, 10 on top and bottom surfaces of the device, the device 1 is simple to put on by the user themselves whilst ensuring that good contact between the device 1 and the user's skin is made.

Furthermore, the portable device 1 is a standalone device in that it does not require any software or an app on a computing device to activate the device 1 or monitor the device 1. This simplifies the device, helping reduce the overall cost of the device 1. In addition, the device 1 does not require setting-up and so the device 1 is ready to be activated as soon as a user wishes to use the device 1.

The portable device 1 is well-suited for long-term monitoring as it can be given to a user with infrequent symptoms to wear for an extended period of time.

The device 1 can also be given to multiple users. The memory within the device 1 is a read-write memory. The device 1 can be given to a first patient to monitor and record their electrical heart signals for a period of observation determined by a medical professional. Once that user no longer needs to use the device 1, the records stored within the memory can be deleted and the device 1 can be given to another user to measure and record their electrical heart signals. This means that the device 1 does not need to be thrown away after a first user no longer needs the device, providing cost benefits in the long run as one device can be used by multiple users. As the device 1 is held against a user when measuring their electrical signals, rather than for example being stuck to a user, the device can be used multiple times.

## Claims

1. A portable device configured to measure activity of a user's heart, the device comprising:
a sensor configured to measure the electrical signals of a user's heart, the sensor comprising:
a first electrode and a second electrode, the first and second electrodes are configured to come into contact with first and second parts of a user's skin, wherein the first and second electrodes are spaced apart from each other and located on opposite sides of the portable device;
an activation switch configured to activate the sensor;
wherein the activation switch is configured to activate the sensor through a single interaction with the activation switch by the user;
wherein once the sensor has been activated, the device is configured to record the measured signals in a memory of the device; and
wherein the sensor is activated for a pre-determined period of time.

2. The portable device of claim 1 wherein the first electrode forms part of the activation switch.

3. The portable device of claim 1 wherein the first electrode is located on a top surface of the device and the second electrode is located on a bottom surface of the device.

4. The portable device of any preceding claim wherein the first electrode is temporarily deformable.

5. The portable device of any preceding claim further comprising a housing configured to house electrical components of the portable device, and wherein the first electrode is integrally formed with the housing.

6. The portable device of any preceding claim wherein the second electrode is detachable.

7. The portable device of any preceding claim wherein the second electrode comprises a raised portion.

8. The portable device of any preceding claim wherein the first and second electrodes are electrically connected to a PCBA via at least one flexible connection.

9. The portable device of any preceding claim further comprising an indication means configured to provide information to a user about the status of the device, wherein the indication means is a form of a light.

10. The portal device of claims 9 comprising an interface, wherein the interface only includes the activation switch and the indication means.

11. The portable device of any preceding claim wherein the device is configured to be held against the user when measuring the electrical signals of a user's heart such that the first part of the user's skin is located on the user's hand and the second part of the user's skin is located anywhere on the user.

12. The portable device of any preceding claim wherein the device is configured to be held against the user when measuring the electrical signals of a user's heart such that the first part of the user's skin is located on the user's hand and the second part of the user's skin is located on the back of the user's wrist.

13. The portable device of any preceding claim further comprising an attachment means configured to allow the user to wear the device.

14. The portable device of any preceding claim further comprising a user-replaceable battery.

15. The portable device of claim 1 wherein the device is configured to record measured signals on-demand, wherein the measured signals comprise a plurality of discrete measurements.

16. The portable device of any preceding claims wherein the device is a multi-use, multi-user device configured to allow recorded measured signals captured by one user to be read and then deleted from the device and then issue the device to another user to allow the device to be used by multiple users at different points of time.
